# EUROPÄISCHE PATENTSCHRIFT

(11) **EP 4 759 248 B1**
(45) Veröffentlichungstag und Bekanntmachung des Hinweises auf die Patenterteilung: **14.10.2026**
(21) Anmeldenummer: 25223087.5
(22) Anmeldetag: 12.12.2025
(51) Int. Cl.: A61B 17/29, A61B 17/00, A61B 18/14

(54) **RASTMECHANISMUS UND HANDGRIFF FÜR EIN CHIRURGISCHES GERÄT MIT EINEM SOLCHEN RASTMECHANISMUS**
LATCHING MECHANISM AND HANDLE FOR A SURGICAL DEVICE WITH SUCH A LATCHING MECHANISM
MÉCANISME D'ENCLIQUETAGE ET POIGNÉE POUR UN INSTRUMENT CHIRURGICAL COMPRENANT UN TEL MÉCANISME D'ENCLIQUETAGE

(30) Priorität: 16.12.2024 DE 102024137928
(43) Veröffentlichungstag der Anmeldung: 17.06.2026
(73) Patentinhaber: BOWA-electronic GmbH & Co. KG, 72810 Gomaringen (DE)
(72) Erfinder: Funder, Marius, 72116 Mössingen (DE); Schiele, Philipp, 72119 Ammerbuch (DE); Izreg, Mahdi, 71034 Böblingen (DE); Dreher, Heike, 72818 Trochtelfingen (DE); Haag, Michael, 72131 Ofterdingen (DE); Burghardt, Martin, 72770 Reutlingen (DE)
(74) Vertreter: Schneider, Peter Christian

(56) Entgegenhaltungen:
- EP-A1- 3 563 780
- EP-A2- 3 970 647
- WO-A1-2022/132779
- DE-A1- 102014 110 881
- DE-U1- 20 305 028
- US-A1- 2009 182 327
- US-A1- 2024 058 053

## Beschreibung

### Gebiet der Erfindung

Die Erfindung betrifft einen Rastmechanismus zur temporären Verrastung eines Mobilelementes mit einem Basiselement, wobei das Mobilelement relativ zum Basiselement auf einer vorgegebenen Bewegungsbahn geführt zwischen einer basisfernen Endstellung und einer basisnahen Endstellung bewegbar, insbesondere schwenkbar, und vorzugsweise in Richtung der basisfernen Endstellung federvorgespannt ist, umfassend
- einen an dem Mobilelement festgelegten und quer zur Bewegungsbahn ausgerichteten Raststift und
- einen bewegbar an dem Basiselement gelagerten Labyrinthkörper mit einem von dem Raststift gleitend umfahrbaren Umlaufkörper mit parallel zu dem Raststift erstreckten Gleitflächen, darunter eine erste und eine zweite Gleitfläche, die zueinander in einem dem Mobilelement zugewandten, spitzen Winkel stehen und an ihren dem spitzen Winkel abgewandten Enden durch eine konkav geformte, dritte Gleitfläche, in die die erste Gleitfläche über eine erste Umlaufkörperkante und die zweite Gleitfläche über eine zweite Umlaufkörperkante übergeht, miteinander verbunden sind,
wobei der Raststift und der Labyrinthkörper ausgelegt sind, derart zusammenzuwirken, dass nach erstmaliger Überführung des Mobilelementes in die basisnahe Endstellung eine Rücküberführung in die basisferne Endstellung blockiert wird und nach nochmaliger Überführung des Mobilelementes in seine basisnahe Endstellung eine Rücküberführung in die basisferne Endstellung wieder zugelassen wird, und wobei der Labyrinthkörper um eine parallel zu dem Raststift ausgerichtete Schwenkachse schwenkbeweglich an dem Basiselement gelagert ist und die Schwenkachse auf der dem Mobilelement abgewandten Seite der dritten Gleitfläche und beabstandet von dieser angeordnet ist.

Die Erfindung betrifft weiter einen Handgriff für ein chirurgisches Gerät, umfassend
- ein Griffgehäuse mit einer Gehäusewandung und einem von der Gehäusewandung umgebenen Gehäuseinnenraum,
- einen relativ zu dem Griffgehäuse verstellbaren Hebel und
- einen Rastmechanismus der vorgenannten Art,

wobei das Griffgehäuse einen das Basiselement des Rastmechanismus tragenden Basiskörper und der Hebel das Mobilelement des Rastmechanismus bilden
und wobei der Labyrinthkörper im Gehäuseinneren gelagert und der Raststift auf einem dem Griffgehäuse zugewandten Vorsprung des Hebels angeordnet ist, welcher wenigstens während eines in Richtung der basisnahen Endstellung letzten Abschnitts der Bewegungsbahn durch eine Ausnehmung in der Gehäusewandung in das Gehäuseinnere hineinragt.

### Stand der Technik

Ein derartiger Rastmechanismus und ein derartiger Handgriff für ein chirurgisches Gerät sind bekannt aus der US 2009/0182327 A1.

Viele chirurgische Geräte, insbesondere endoskopische chirurgische Geräte, zeichnen sich durch einen am distalen Ende eines Schaftes angebrachten Effektor aus, der mittels eines am proximalen Ende des Schaftes angeordneten und mit Funktionselementen versehenen Handgriffs betätigbar ist. Die am Handgriff angebrachten Funktionselemente sind meist mechanischer und/oder elektrischer Natur. Der Schaft ist mit entsprechenden elektrischen und/oder mechanischen Verbindungselementen ausgestattet. Es sind Effektoren mit unterschiedlichsten Funktionen, beispielweise Greif-, Schneid-, Strombeaufschlagungs- oder sensorischen Funktionen bekannt. Häufig sind mehrere solcher Funktionen in einem Effektor kombiniert. Insbesondere ist bei einer Vielzahl von Effektoren eine Greiffunktion realisiert. Hierzu weist der Effektor zwei relativ zueinander verschwenkbare Klemmbacken auf, von denen wenigstens eine über ein im Schaft geführtes Gestänge mit einem an einem feststehenden Griff angeordneten Betätigungshebel verbunden ist. Bei dem Hebel kann es sich insbesondere um einen Schwenk- oder Schiebehebel handeln. In jedem Fall ist er relativ zu dem feststehenden Griff auf einer vorgegebenen Bewegungsbahn geführt bewegbar, wobei häufig eine pistolenartige, einhändig bedienbare Konfiguration realisiert ist.

Aus der EP 3 970 647 A2, der US 2024/0058053 A1 und der EP 3 563 780 A1 ist ein derartiges chirurgisches Instrument mit einem als Greifer ausgebildeten Effektor bekannt, der mittels eines Schwenkhebels betätigbar ist, welcher seinerseits an einem den feststehenden Griff bildenden, hohlen Griffgehäuse angelenkt ist. Der Hebel ist insbesondere zwischen einer gehäusefernen Endstellung, die er insbesondere im losgelassenen Zustand einnimmt, und einer gehäusenahen Endstellung, die er insbesondere im angezogenen Zustand einnimmt, hin und her verschwenkbar und in Richtung seiner gehäusefernen Endstellung federvorgespannt. Das Anziehen des Hebels, d. h. seine Verschwenkung in die gehäusenahe Endstellung, führt zu einem Schließen der Klemmbacken des Effektors. Umgekehrt führt ein Loslassen des Hebels, d. h. seine Überführung in die gehäuseferne Endstellung, zu einem Öffnen der Klemmbacken. Häufig ist es erwünscht, den Hebel wenigstens temporär in seiner gehäusenahen Endstellung und damit die Klemmbacken in ihrer Klemmstellung zu fixieren. In einem solchen Zustand kann der Chirurg seine Finger zur Betätigung weiterer Funktionselemente am Handgriff benutzen, ohne dass der Halt einer zwischen den Klemmbacken geklemmten Struktur, beispielsweise eines Abschnitts biologischen Gewebes, verloren geht.

Zum Zwecke einer solchen temporären Fixierung offenbaren die genannten Druckschriften einen Rastmechanismus zur temporären Verrastung des Hebels mit dem Griffgehäuse. Der Hebel entspricht dabei dem Mobilelement des Rastmechanismus. Das Griffgehäuse könnte unmittelbar dem Basiselement des Rastmechanismus entsprechen; bei der vorbekannten Gestaltung entspricht es jedoch einem übergeordneten Basiskörper, an dem, wie weiter unten noch näher erläutert, das eigentliche Basiselement verstellbar gelagert ist. Der Hebel weist einen dem Griffgehäuse zugewandten Vorsprung auf, der beim Anziehen des Hebels durch eine Ausnehmung in der Gehäusewandung in das Gehäuseinnere eintaucht. An der Spitze des Vorsprungs ist ein quer zu dessen und damit auch quer zu seiner eigenen Bewegungsbahn ausgerichteter Raststift fixiert. Dieser kollidiert im Gehäuseinneren mit einem im Wesentlichen dreieckig geformten, hier aus nachfolgend noch näher zu erläuternden Gründen als Umlaufkörper bezeichneten Bereich einer hier verallgemeinernd als Labyrinthkörper angesprochenen Rastplatte. Insbesondere weist der Umlaufkörper eine erste und eine zweite Gleitfläche auf, die gemeinsam einen spitzen Winkel einschließen, welcher dem anlaufenden Raststift entgegengerichtet ist. An ihren dem Raststift abgewandten Enden sind die erste und zweite Gleitfläche über eine dritte Gleitfläche miteinander verbunden, die eine konkave Krümmung aufweist. Dieser Umlaufkörper ist auf einer entlang der Griffachse des Griffgehäuses linear verschiebbaren Platte fixiert, die bei der bei der Vorrichtung gemäß EP 3 970 647 A2 und US 2024/0058053 A1 mittels einer bidirektional wirkenden Ringfeder und bei der bei der Vorrichtung gemäß der EP 3 563 780 A1 mittels einer Schraubenfeder in einer Initialstellung gegenüber einem Basiselement gehalten wird. In besagter Initialstellung der Rastplatte liegt die Spitze des Umlaufkörpers in Richtung deren linearer Verschiebbarkeit geringfügig versetzt zur Bewegungsbahn des Raststiftes. Bei der Kollision gleitet dieser daher entlang der ersten Gleitfläche und erzwingt dadurch eine Verschiebung der Rastplatte aus ihrer Initialstellung gegen die Kraft der Feder. Sobald der Raststift das hintere Ende der ersten Gleitfläche erreicht hat, rutscht er über die erste Umlaufkörperkante und die Feder zieht die Rastplatte und damit auch den Umlaufkörper wieder in Richtung der Initialstellung. Die Rückstellung erfolgt jedoch nicht vollständig, da der Raststift zuvor jenseits des Scheitelpunktes der konkaven, dritten Gleitfläche an dieser anschlägt und eine weitere Rückstellung unterbindet. Beim nachfolgenden Loslassen des Hebels drückt die auf diesem lastende Federvorspannung den Raststift in den Scheitelpunkt der dritten Gleitfläche bzw. verschiebt die Rastplatte entsprechend. Eine selbsttätige Rücküberführung des Hebels in seine gehäuseferne Endstellung ist damit genauso unterbunden wie eine selbsttätige Rückstellung der Rastplatte in ihre Initialstellung. Erneute Betätigung des Hebels, d. h. seine erneute Überführung aus der oben erläuterten, nur geringfügig von der gehäusenahen Endstellung entfernten Raststellung in die gehäusenahe Endstellung, erlaubt es der Feder, die Rastplatte und damit auch den Umlaufkörper wieder weiter in Richtung der Initialstellung zu ziehen, wobei der Raststift aus dem Scheitelpunkt der Krümmung der dritten Gleichfläche zur zweiten Umlaufkörperkante gleitet, über welche die dritte mit der zweiten Gleitfläche verbunden ist. Sobald der Raststift diese zweite Umlaufkörperkante überwunden hat, kann der Hebel seiner Federvorspannung folgend in seine gehäuseferne Endstellung verschwenken. Der Raststift gleitet dabei über die zweite Gleitfläche in Richtung der Spitze des Umlaufkörpers. Die zweite Gleitfläche ist so geformt, dass dabei die Rastplatte unter umgekehrter Auslenkung der Feder über die Initialstellung hinaus gezwungen wird. Erst wenn der Hebel soweit verschwenkt ist, dass der Raststift über die Spitze des Umlaufkörpers gleitet, d.h. wenn der Umlaufkörper von dem Raststift vollständig umlaufen ist, schnappt die Rastplatte in ihre Initialstellung zurück, sodass eine erneute (dritte) Betätigung des Hebels den oben geschilderten Vorgang erneut starten kann. Die genannte Druckschrift nennt zwar auch die Möglichkeit einer schwenkbaren Anlenkung der Rastplatte am Basiselement, gibt hierzu aber keinerlei für den Fachmann nacharbeitbare Hinweise zur Umsetzung. Solche finden sich jedoch in der eingangs genannten, gattungsbildenden US 2009/0182327 A1.

Bei den speziellen, in der EP 3 970 647 A2 und der EP 3 563 780 A1 geschilderten Gestaltungen ist die Rastplatte auf einer Selektorplatte gelagert und mittels der Ring- bzw. Schraubenfeder an dieser fixiert. Die Selektorplatte ihrerseits ist linear verschieblich im Griffgehäuse gelagert und kann dort insbesondere zwei unterschiedliche Selektorstellungen einnehmen. In einer ersten Selektorstellung ist der Umlaufkörper auf der Bewegungsbahn des Raststiftes positioniert, sodass die oben geschilderte Wechselwirkung stattfinden kann. In der zweiten Selektorstellung ist der Umlaufkörper deutlich außerhalb der Bewegungsbahn des Raststiftes positioniert, sodass Raststift und Umlaufkörper bei der Betätigung des Hebels nicht miteinander kollidieren, d.h. die erläuterte Rastfunktion "ausgeschaltet" ist.

Das Funktionieren der aus der EP 3 970 647 A2, der US 2024/0058053 A1 und der EP 3 563 780 A1 vorbekannten Rastmechanismen hängt stark von der präzisen Funktion der Ring- bzw. Schraubenfeder ab, die im Rahmen der Wechselwirkung zwischen Raststift und Umlaufkörper in zwei unterschiedliche Richtungen ausgelenkt wird und im kräftefreien Zustand eine vorbestimmte Initialstellung des Labyrinthkörpers zuverlässig gewährleisten muss. Dies erlaubt nur sehr geringe Toleranzen bei Fertigung und Betrieb. Selbst wenn durch aufwändige und teure Qualitätssicherung bei der Herstellung Fertigungstoleranzen weitgehend vermieden werden können, können doch alterungs- und temperaturbedingte Schwankungen eine präzise Funktion behindern. Beim Rastmechanismus gemäß US 2009/0182327 A1 wird der dortige, kippbar gelagerte Labyrinthkörper mittels einer in analoger Weise bidirektional wirkenden Blattfeder vorgespannt bzw. rückgestellt, was zu analogen Problemen führt.

Die DE 10 2024 110 881 A1 offenbart ein Chirurgisches Instrument mit einem Handgriff, an dem zwei vorzugsweise hebelartige Handhaben gegeneinander schwenkbar gelagert sind, welche in einer vorzugsweise zusammengeklappten Schwenkposition mittels einer Umlaufsperre verrastbar sind, bestehend aus einem eine Kulissenführung aufweisendes Kulissenelement an einer der Handhaben und einem Nockenelement an einer anderen der Handhaben das einen mit der Kulissenführung in Führungs- und Rasteingriff bringbaren Umlaufnocken hat, von denen das Kulissenelement und/oder das Nockenelement schwenkbar an der jeweiligen Handhabe gelagert sind und der Umlaufnocken und/ oder die Kulissenführung mittels wenigstens einer Feder vorgespannt ist, um die Umlaufnocke und/oder die Kulissenführung in Abhängigkeit der aktuellen Betätigung der Handhaben in eine definierte Kulissen-Umlaufrichtung anzutreiben, wobei die wenigstens eine Feder eine Federkraft senkrecht zur KulissenUmlaufrichtung auf die Umlaufnocke und/oder die Kulissenführung ausübt und die Umlaufnocke und/oder die Kulissenführung Abgleitebenen aufweist, welche bei deren Übergleiten den Aufbau der Federkraft bewirken und nach deren Übergleiten eine rückwärtige Bewegung des Umlaufnocken n der Führungskulisse blockieren.

### Aufgabenstellung

Es ist die Aufgabe der vorliegenden Erfindung, einen gattungsgemäßen Rastmechanismus bzw. einen gattungsgemäßen Handgriff für ein chirurgisches Gerät derart weiterzubilden, dass eine günstigere Herstellung, eine zuverlässigere Funktion und/oder ein geringerer Bauraum ermöglich werden.

### Darlegung der Erfindung

Diese Aufgabe wird in Verbindung mit den Merkmalen des Oberbegriffs von Anspruch 1 durch zwei antagonistisch auf den Labyrinthkörper einwirkende Federn, deren einander entgegengesetzte Drehmomente den Labyrinthkörper, wenn er sich nicht in Wechselwirkung mit dem Raststift befindet, in einer vorgegebenen Initialstellung halten, gelöst.

Bevorzugte Ausführungsformen sind Gegenstand der abhängigen Ansprüche.

Die Erfindung greift den vorbekannten Gedanken einer Verschwenkbarkeit des Labyrinthkörpers anstelle seiner lediglich linearen Verschiebbarkeit auf. Insbesondere wird die Verschwenkbarkeit des Labyrinthkörpers durch eine speziell angeordnete und ausgerichtete Schwenkachse realisiert. Der Begriff "Schwenkachse" ist hier rein funktional zu verstehen. Räumlich-körperliche Realisierungsmöglichkeiten können beispielsweise in Lagermulden gelagerte Achsstutzen oder eine Starrachse, die eine Hülse durchsetzt, umfassen. Die Schwenkachse ist zum einen parallel zu dem Raststift ausgerichtet. Zum anderen ist sie beabstandet zu dem von den drei Gleitflächen berandeten Umlaufkörper angeordnet, nämlich auf der dem spitzen Winkel bzw. dem anlaufenden Raststift abgewandten Seite der konkaven, dritten Gleitfläche. Hierdurch wird eine Nickbewegung des gesamten Umlaufkörpers ermöglich, ohne dass ein Absenken eines Teils des Umlaufkörpers zu einem Anheben eines anderen Teiles führen würde, wie dies der Fall wäre, wenn die Schwenkachse durch den Umlaufkörper verlaufen würde. Bei theoretisch unendlich großem Abstand zwischen Schwenkachse und dritter Gleitfläche entspräche diese Nickbewegung der vorbekannten Linearbewegung des Umlaufkörpers; bei einem praktisch realisierbaren, endlichen Abstand im Inneren des Griffgehäuses kommt die Nickbewegung der vorbekannten Linearbewegung zumindest nahe, sodass insoweit auf die obige Erläuterung zur Funktionsweise verwiesen werden kann. Allerdings vermeidet die vorliegende Erfindung die Probleme, die mit einer echten Linearführung und der in diesem Zusammenhang erforderlichen, linear gerichteten Federwirkung verbunden sind, indem sie diese durch einfacher realisierbare Rotationsbewegungen bzw. -führungen und Drehmomente ersetzt. Ein entsprechender, erfindungsgemäßer Rastmechanismus ist daher günstiger in der Herstellung und robuster gegenüber Schwankungen durch Fertigungstoleranzen, Alterung oder Temperaturunterschiede.

Erfindungsgemäß ist vorgesehen, dass der Labyrinthkörper durch zwei antagonistisch auf ihn einwirkende Federn zumindest dann, wenn er sich nicht in Wechselwirkung mit dem Raststift befindet, in einer vorgegebenen Initialstellung gehalten wird. Die Federn üben einander entgegengesetzte Drehmomente auf den Labyrinthkörper aus. Dabei ist es nicht erforderlich, dass beide Federn in allen Schwenkpositionen des Labyrinthkörpers auf diesen einwirken. Vielmehr ist es möglich und bevorzugt vorgesehen, dass zumindest in einigen Schwenkpositionen nur eine der Federn auf den Labyrinthkörper einwirkt. Zumindest aber in der Nähe der Initialstellung sollten beide Federn wirksam sein, um durch ihre entgegengesetzten Wirkungen besagte Initialstellung präzise zu definieren. Besonders günstig ist es, wenn besagte Nähe zur Initialstellung durch eine besonders starke Abhängigkeit der ausgeübten Federkraft wenigstens einer der Federn vom Verschwenkungswinkel des Labyrinthkörpers definiert ist. Insbesondere kann vorgesehen sein, dass das von dieser Feder auf den Labyrinthkörper ausgeübte Drehmoment einerseits der Initialstellung größer und andererseits der Initialstellung kleiner als das von der anderen Feder entgegengesetzt auf den Labyrinthkörper ausgeübte Drehmoment ist. Je größer die Steigung der Abhängigkeit des erzeugten Drehmomentes vom Verschwenkungswinkel ist, desto präziser ist die Initialstellung des Labyrinthkörpers definiert. Besagte Initialstellung kann auch zum Zwecke der nachfolgenden Funktionsbeschreibung als Ruhelage oder initiale Winkelstellung des Labyrinthkörpers bezeichnet werden. Diese ist vorzugsweise so ausgelegt, dass der sich bei Betätigung des Hebels nähernde Raststift in unmittelbarer Nähe der Spitze des Umlaufkörpers mit der ersten Gleitfläche kollidiert.

Bei weiterem Vorschub des Raststiftes gleitet dieser auf der ersten Gleitfläche entlang und erzwingt dadurch eine Nickbewegung des gesamten Labyrinthkörpers um die Schwenkachse.

Damit besagte Nickbewegung kontrolliert erfolgen kann, verläuft sie bevorzugt entgegen der Kraftwirkung einer der Federn, insbesondere entgegen der Kraftwirkung der bei dieser Winkelstellung stärkeren Feder. Bei einer besonders bevorzugten Ausführungsform ist diese als eine Schenkelfeder ausgebildet. Diese weist einen ersten Schenkel, einen zweiten Schenkel und eine die Schenkel verbindende Federöse auf und stützt sich zwischen dem Basiselement und dem Labyrinthkörper ab, wobei die Federöse einen Lagerzapfen der Schwenkachse konzentrisch umläuft, der erste Schenkel am Basiselement festgelegt ist und der zweite Schenkel am Labyrinthkörper festgelegt ist. Als Lagerzapfen kann z.B. ein Achsstutzen des Labyrinthkörpers oder ein Abschnitt einer von einer Starrachse durchsetzten Lagerhülse dienen. In jedem Fall lässt sich hierdurch während der Nickbewegung eine dieser Nickbewegung entgegengerichtete Federkraft erzeugen, die die erste Gleitfläche gegen den auf ihr gleitenden Raststift drückt, sodass eine kontrollierte Bewegung gewährleistet ist.

Die andere Feder übt in dieser Phase ein deutlich geringeres, vorzugsweise überhaupt kein Drehmoment auf den Labyrinthkörper aus. Sie ist bevorzugt als eine an dem Basiselement festgelegte Federzunge ausgebildet. Ihr freies Ende liegt zumindest in einigen Schwenkpositionen an einer Anlagefläche des Labyrinthkörpers an und übt ein der Federwirkung der Schenkelfeder entgegengerichtetes Drehmoment auf den Labyrinthkörper aus. In der hier beschriebenen Phase liegt sie jedoch bevorzugt gar nicht oder höchstens in einer nur ein geringes Drehmoment erzeugenden Weise an der Anlagefläche des Labyrinthkörpers an.

In dem Moment, in dem der Raststift das Ende der ersten Gleitfläche, d. h. die erste Umlaufkörperkante erreicht hat, bewirkt die aufgebaute Federkraft, insbesondere der Schenkelfeder, ein Zurücknicken des Labyrinthkörpers in Richtung seiner Initialstellung. Bei einem nachfolgenden Bewegen des Mobilelementes in Richtung seiner basisfernen Endstellung nähert sich der Raststift "von hinten" dem Labyrinthkörper, insbesondere dessen dritter Gleitfläche an und kann in den Scheitelpunkt der konkaven, dritten Gleitfläche rutschen, sodass das Mobilelement gegenüber dem Basiselement, mithin der Hebel des chirurgischen Gerätes gegenüber dessen Griff, verrastet ist.

Erneute Betätigung des Hebels erlaubt ein weiteres, von der Federvorspannung insbesondere der Schenkelfeder getriebenes Zurücknicken des Labyrinthkörpers, wobei der Raststift weiter über die konkave, dritte Gleitfläche gleitet, bis er deren zweites Ende, d.h. die zweite Umlaufkörperkante und damit den Übergang zur zweiten Gleitfläche erreicht hat.

Eine Rücküberführung des Mobilelementes in seine basisferne Endstellung, insbesondere des Hebels in seine gehäuseferne Endstellung, ist nun wieder möglich. Dabei gleitet der Raststift an der zweiten Gleitfläche entlang und setzt das Zurücknicken des Labyrinthkörpers über dessen Initialstellung hinaus fort. Spätestens hier setzt die Wirkung der Federzunge (wieder) ein. Das dadurch erzeugte Drehmoment wirkt einem weiteren Zurücknicken des Labyrinthkörpers entgegen und sorgt für eine zuverlässige Anlage des Raststiftes an der zweiten Gleitfläche.

In dem Moment, in dem der Raststift das Ende der zweiten Gleitfläche, mithin die Spitze des Umlaufkörpers erreicht hat, wirken die einander entgegengesetzten Kräfte der Schenkelfeder einerseits und der Federzunge andererseits einander unmittelbar entgegen. Um sicherzustellen, dass in dieser Situation die initiale Winkelstellung des Labyrinthkörpers wieder erreicht wird, ist bevorzugt vorgesehen, dass die Federkraft der Federzunge diejenige der Schenkelfeder überwiegt - und zwar vorzugsweise deutlich. Die Federzunge sorgt dafür, dass der Labyrinthkörper mindestens so weit nickt, bis sie selbst wieder wirkungslos ist, d. h. das durch sie auf den Labyrinthkörper ausgeübte Drehmoment wieder geringer ist als von der Schenkelfeder ausgeübte Drehmoment. Die entgegengesetzte Kraft der Schenkelfeder kann dann nämlich dafür sorgen, dass der Labyrinthkörper nicht weiter nickt, sondern in seiner Initialstellung verbleibt. Bei deutlichem Kräfteunterschied der beiden Federn lässt sich die initiale Winkelstellung also präzise einstellen und zwar unabhängig von Fertigungstoleranzen oder Alterungs- und Temperatureinflüssen. Als Faustregel gilt dabei, je größer der Kräfteunterschied zwischen den beiden Federn ist, desto robuster ist die initiale Winkelstellung gegen schädliche Einflüsse.

Ein kritischer Moment im Rahmen der oben beschriebenen Wechselwirkung ist derjenige Zeitpunkt, zu dem der Raststift die erste Umlaufkörperkante zwischen erster und dritter Gleitfläche erreicht, d. h. der Moment, zu dem die Rücknickbewegung des Labyrinthkörpers einsetzt. Bei unglücklichem Timing könnte es passieren, dass der Labyrinthkörper so schnell zurücknickt, dass der Raststift nicht im Scheitelpunkt der konkaven, dritten Gleitfläche zu liegen kommt, sondern den Umlaufkörper ohne Wechselwirkung mit der dritten Gleitfläche an sich vorbeischwenken lässt und sogar die zweite Umlaufkörperkante zwischen dritter und zweiter Gleitfläche verpasst. Die gewünschte Rastwirkung käme in diesem Fall nicht zustande. Um dies zuverlässig zu verhindern, ist bei einer Weiterbildung der Erfindung vorgesehen, dass der Labyrinthkörper weiter einen vom Umlaufkörper beabstandeten, insbesondere in Richtung der Schwenkachse beabstandeten der dritten Gleitfläche gegenüber liegenden Auffangsteg aufweist. Dadurch wird einem unkontrollierten Zurücknicken des Labyrinthkörpers entgegengewirkt. Besagter Auffangsteg schlägt nämlich beim Zurücknicken des Labyrinthkörpers am Raststift an und stoppt zunächst das weitere Zurücknicken. Erst wenn das Mobilelement bewusst freigegeben bzw. in Richtung seiner basisfernen Endstellung bewegt wird, kommt der Raststift vom Auffangsteg frei und befindet sich dann zuverlässig in einer Position, in der die konkave dritte Gleitfläche, insbesondere die zweite Umlaufkörperkante beim weiteren Zurücknicken des Labyrinthkörpers zwingend an ihm hängenbleibt. Weitere Freigabe des Mobilelementes führt dann zu der angestrebten Positionierung des Raststiftes im Scheitelpunkt der konkaven, dritten Gleitfläche und somit zur gewünschten Rastwirkung.

Auch bei der nachfolgenden, die Verrastung wieder lösenden Betätigung des Hebels spielt der Auffangsteg eine Rolle. Er bildet mit dem der zweiten Umlaufkörperkante benachbarten Bereich der dritten Gleitfläche einen Kanal, welcher den Raststift dazu zwingt, entlang der dritten Gleitfläche zur zweiten Umlaufkörperkante und über diese hinaus zu gleiten, sodass der Raststift aus dem Labyrinth freikommt.

Aus Gründen einer symmetrischen Kräfteverteilung ist bevorzugt vorgesehen, dass der Labyrinthkörper zwei zueinander parallele, voneinander um einen Spalt beabstandete und hinsichtlich der ersten, zweiten und dritten Gleitflächen gleich gestaltete Umlaufkörper umfasst und der Raststift sich beidseitig eines Vorsprungs des Mobilelementes erstreckt, welcher in der basisnahen Endstellung in den Spalt zwischen den Umlaufkörpern eingreift. Mit anderen Worten sind also zwei im Wesentlichen gleich gestaltete Umlaufkörper vorgesehen, die mit jeweils einem Abschnitt des Raststiftes in gleicher Weise wechselwirken. Diese symmetrische Kraftverteilung verhindert das Auftreten von unerwünschten Kippmomenten, die im ungünstigsten Fall zu einer Verkeilung des Mechanismus führen könnten.

Für den Auffangsteg ist eine derartige Zweiteilung, die die Herstellung durchaus verkompliziert, nicht erforderlich. Daher ist bevorzugt vorgesehen, dass den beiden Umlaufkörpern ein gemeinsamer, sich über die gemeinsame Breite der Umlaufkörper und des Spaltes erstreckender Auffangsteg gegenüberliegt. Insbesondere bei Realisierungen des erfindungsgemäßen Rastmechanismus in Kunststoffspritzgusstechnik sollten filigrane Gestaltungen nach Möglichkeit vermieden werden, um den Formenbau nicht unnötig zu verkomplizieren und damit zu verteuern.

Weitere Einzelheiten und Vorteile der Erfindung ergeben sich aus der nachfolgenden speziellen Beschreibung und den Zeichnungen.

### Kurzbeschreibung der Zeichnungen

Es zeigen:
- Figur 1:: eine perspektivische Ansicht eines erfindungsgemäßen chirurgischen Gerätes,
- Figur 2:: eine teilweise geöffnete, perspektivische Darstellung eines erfindungsgemäßen Handgriffs,
- Figur 3:: eine Detaildarstellung einer Ausführungsform des erfindungsgemäßen Rastmechanismus,
- Figur 4:: eine Detaildarstellung einer bevorzugten Ausführungsform des erfindungsgemäßen Rastmechanismus,
- Figur 5:: eine erste perspektivische Darstellung einer bevorzugten Ausführungsform eines Labyrinthkörpers des erfindungsgemäßen Rastmechanismus,
- Figur 6:: eine zweite perspektivische Darstellung des Labyrinthkörpers von Figur 5,
- Figur 7:: eine Schnittdarstellung durch den Rastmechanismus von Figur 4 in einer ersten Betätigungsstellung,
- Figur 8:: eine Schnittdarstellung durch den Rastmechanismus von Figur 4 in einer zweiten Betätigungsstellung,
- Figur 9:: eine Schnittdarstellung durch den Rastmechanismus von Figur 4 in einer dritten Betätigungsstellung,
- Figur 10:: eine Schnittdarstellung durch den Rastmechanismus von Figur 4 in einer vierten Betätigungsstellung,
- Figur 11:: eine Schnittdarstellung durch den Rastmechanismus von Figur 4 in einer fünften Betätigungsstellung,
- Figur 12:: eine Schnittdarstellung durch den Rastmechanismus von Figur 4 in einer sechsten Betätigungsstellung,
- Figur 13:: eine Schnittdarstellung durch den Rastmechanismus von Figur 4 in einer siebten Betätigungsstellung,
- Figur 14:: eine Schnittdarstellung durch den Rastmechanismus von Figur 4 in einer achten Betätigungsstellung,
- Figur 15:: eine Schnittdarstellung durch den Rastmechanismus von Figur 4 in einer neunten Betätigungsstellung,
- Figur 16:: eine Schnittdarstellung des erfindungsgemäßen Rastmechanismus im ausgeschalteten Zustand in der basisfernen Endstellung des Mobilelementes sowie
- Figur 17:: eine Schnittdarstellung des erfindungsgemäßen Rastmechanismus im ausgeschalteten Zustand in der basisnahen Endstellung des Mobilelementes.

### Beschreibung bevorzugter Ausführungsformen

Gleiche Bezugszeichen in den Figuren deuten auf gleiche oder analoge Elemente hin.

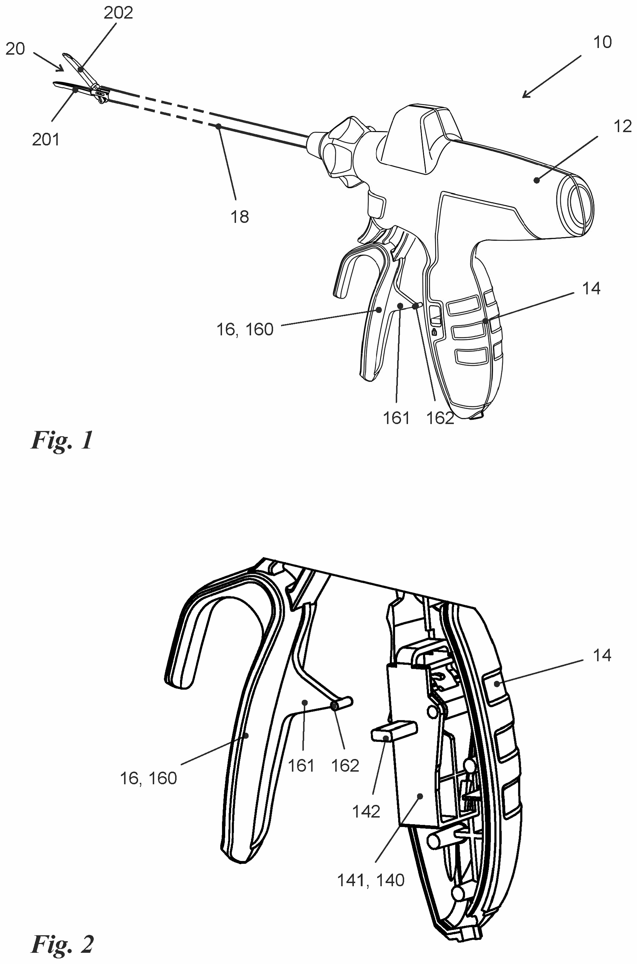

Figur 1 zeigt eine perspektivische Darstellung einer rein beispielhaften Ausführungsform eines erfindungsgemäßen chirurgischen Gerätes 10. Das chirurgische Gerät 10 umfasst einen proximalen Bedienteil 12, der insbesondere ein starres Griffgehäuse 14 und einen demgegenüber schwenkbeweglich angelenkten Hebel 16 aufweist. Von dem Bedienteil 12 erstreckt sich ein Schaft 18, an dessen distalem Ende ein Effektor 20 angebracht ist. Der Effektor 20 umfasst zwei Klemmbacken 201, 202, von denen eine erste Klemmbacke 201 starr am Schaft 18 fixiert und eine zweite Klemmbacke 202 schwenkbeweglich am Schaft 18 angelenkt ist. Die zweite Klemmbacke 202 ist über ein im Schaft 18 verlaufendes, mechanisches Gestänge mit dem Hebel 16 wirkverbunden, sodass eine Betätigung, d.h. Verschwenkung des Hebels 16 zu einer entsprechenden Verschwenkung der zweiten Klemmbacke 202 führt. Insbesondere kann durch Anziehen des Hebels 16, d.h. Verschwenkung in Richtung auf das Griffgehäuse 14, ein Gewebe eines Patienten zwischen den Klemmbacken 101, 102 geklemmt werden.

Auf seiner dem Griffgehäuse 14 zugewandten Seite weist der Hebel 16 einen Vorsprung 161 auf, dessen Spitze einen quer zur Verschwenkungsrichtung stehenden Raststift 162 trägt. Der Vorsprung 161 und der Raststift 162 sind in der vergrößerten Darstellung von Figur 2 besser erkennbar.

Im Inneren des Gehäuses 14 befindet sich ein linear verstellbarer Selektorkörper 141, der mittels eines die Gehäusewandung durchdringenden Schiebers 142 zwischen einer "eingeschalteten" und einer "ausgeschalteten" Stellung verschiebbar ist, auf die weiter unten noch näher eingegangen werden soll. Im Kontext des nachfolgend zu beschreibenden, erfindungsgemäßen Rastmechanismus bilden die Selektorkörper 141 das "Basiselement" 140 und der Hebel 16 das "Mobilelement" 160. Denkbar sind jedoch auch Ausführungsformen, bei denen kein gesonderter Selektorkörper 141 vorgesehen ist und das Griffgehäuse 14 selbst das "Basiselement" 140 des Rastmechanismus bildet.

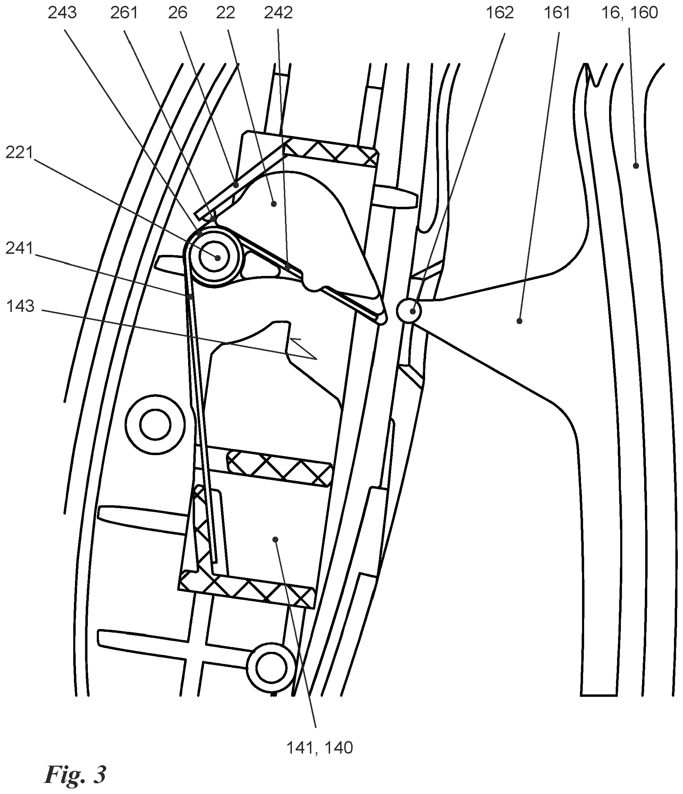

Figur 3 zeigt den "eingeschalteten" erfindungsgemäßen Rastmechanismus in einer Stellung, in der das Mobilelement 160 bzw. der Hebel 16 auf das Basiselement 140 bzw. den Selektorkörper 141 zubewegt wird, seine basis- bzw. gehäusenahe Endstellung aber noch nicht erreicht hat. Das Griffgehäuse 14 ist in Figur 3 geöffnet dargestellt, um einen Blick auf das Basiselement 140 bzw. den Selektorkörper 141 zu gewähren. Dieses bzw. dieser befindet sich in der in Figur 3 dargestellten Stellung in seiner "eingeschalteten" Position, welche die nachfolgend zu erläuternde Wechselwirkung mit dem Mobilelement 160 bzw. dem Hebel 16, insbesondere dem Raststift 162 erlaubt.

Wesentlicher basisseitiger Bestandteil des erfindungsgemäßen Rastmechanismus ist der schwenkbeweglich am Basiselement 140 bzw. dem Selektorkörper 141 angelenkte Labyrinthkörper 22, der den unmittelbaren Wechselwirkungspartner für den Raststift 162 darstellt. Wie in Figur 3 gut erkennbar, ist der Labyrinthkörper 22 durch zwei antagonistisch wirkende Federn 24, 26 vorgespannt. Zum einen ist eine Schenkelfeder 24 vorgesehen, deren erster Schenkel 241 am Basiselement 140 bzw. am Selektorkörper 141 festgelegt ist und deren zweiter Schenkel 242 am Labyrinthkörper 22 festgelegt ist. Die Federöse 243, über welche die beiden Schenkel 241, 242 miteinander verbunden sind, ist konzentrisch um einen Achsstutzen 221 gelagert, welcher die Schwenkachse des Labyrinthkörpers 22 definiert. Die in der in Figur 3 gezeigten Initialstellung des Labyrinthkörpers 22 auf Druck belastete Schenkelfeder 24 übt also ein in Figur 3 entgegen dem Uhrzeigersinn gerichtetes Drehmoment auf den Labyrinthkörper 22 aus. Weiter ist eine vorzugsweise einstückig mit dem Basiselement 140 bzw. dem Selektorkörper 141 verbundene, in der gezeigten Ausführungsform als Federzunge 26 ausgebildete Druckfeder vorgesehen, die senkrecht zur Schwenkachse ausgerichtet ist und benachbart zu dieser mit einem Anlagepunkt 261 am Labyrinthkörper anliegt. In Figur 3 unterhalb des Anlagepunktes 261 erstreckt sich die Außenfläche des Labyrinthkörpers 22 konzentrisch zur Schwenkachse. In Figur 3 oberhalb des Anlagepunktes 261 steht die Außenfläche des Labyrinthkörpers 22 tangential ab. Dies führt dazu, dass eine Verschwenkung des Labyrinthkörpers im Gegenuhrzeigersinn zu einem sich steigernden Gegen-Drehmoment der Federzunge 26 führt. Da die Federzunge 26 jedoch deutlich stärker ausgebildet ist als die Schenkelfeder 24 ist letztere nicht in der Lage, ein für eine solche Verschwenkung hinreichend starkes Drehmoment aufzubringen. Andererseits führt eine Verschwenkung des Labyrinthkörpers 22 im Uhrzeigersinn dazu, dass sich der Anlagepunkt 261 der Federzunge 26 auf den konzentrischen Teil der Achskörper-Außenfläche verlagert, wo die Kraftwirkung der Federzunge 26 zu keinerlei Drehmoment führt. Bei Ausführungsformen mit nur geringem Federweg der Federzunge 26 kann deren Anlagepunkt 261 auch ganz vom Labyrinthkörper 22 abheben. Einer solchen Verschwenkung kann die Schenkelfeder 24 also ohne weiteres entgegenwirken. Im Ergebnis stellt sich die in Figur 3 gezeigte Initialstellung des Labyrinthkörpers 22 als dessen stabile Gleichgewichts- bzw. Ruhelage ein. Die in Figur 3 dargestellte Konstellation zeigt also die stabile Ausgangskonstellation für die nachfolgend zu beschreibende Funktionsweise des erfindungsgemäßen Rastmechanismus.

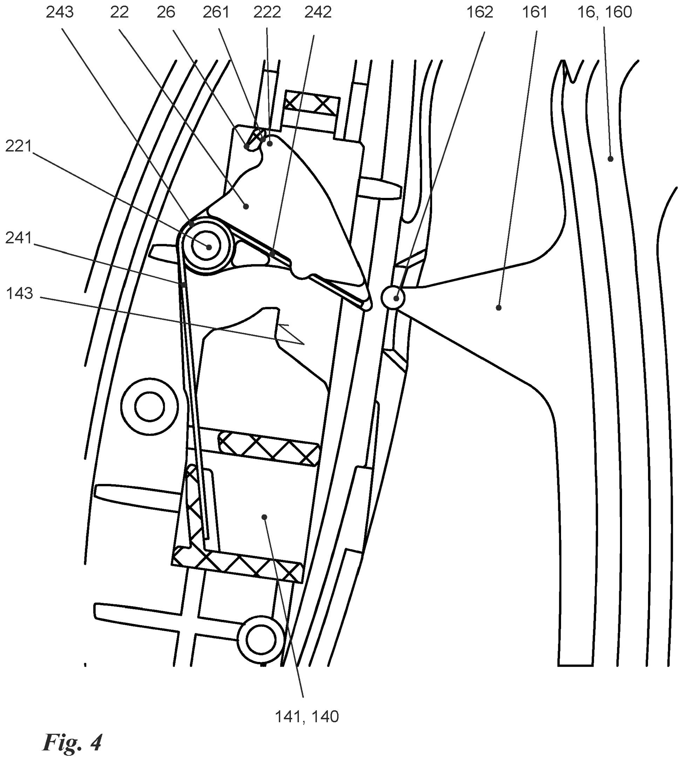

Figur 4 zeigt eine alternative Gestaltung, bei der die Federzunge 26 parallel zur Schwenkachse ausgerichtet ist. dies erlaubt es, bei gleichem Bauraum einen größeren Abstand zur Schwenkachse einzuhalten und dadurch bei gleicher Federkraft ein höheres Drehmoment auf den Labyrinthkörper 22 auszuüben. Bei der gezeigten Ausführungsform weist der Labyrinthkörper 22 einen besonderen Ausleger 222 zur Wechselwirkung mit der Federzunge 26 auf. Dies stellt die bevorzugte Ausführungsform der Erfindung dar.

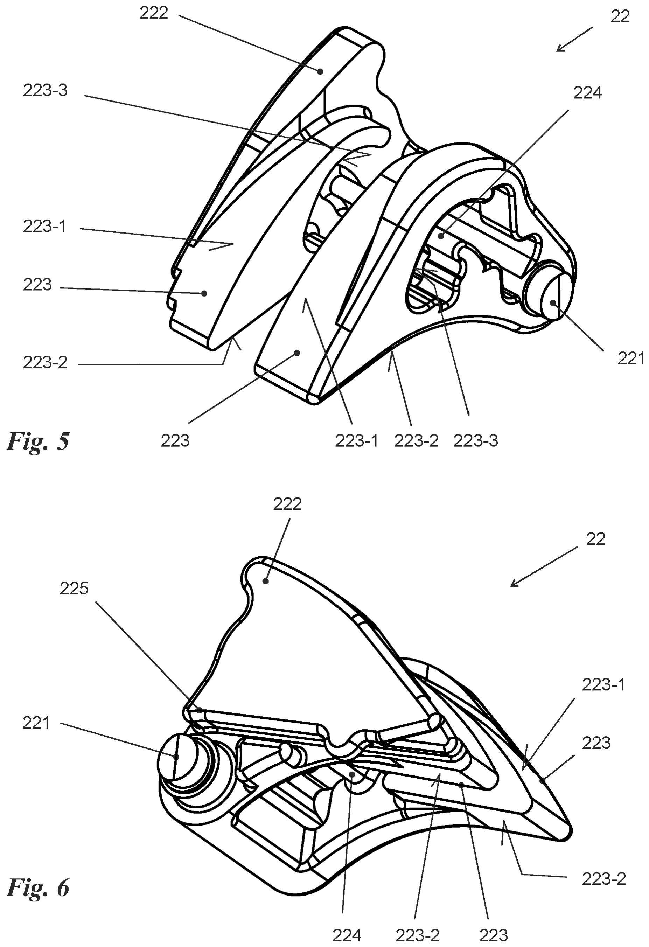

Die Figuren 5 und 6 zeigen eine bevorzugte Ausführungsform des Labyrinthkörpers 22 in zwei unterschiedlichen, perspektivischen Darstellungen. Bei der gezeigten Ausführungsform besteht der Labyrinthkörper 22 im Wesentlichen aus zwei weitgehend gleich gestalteten und parallel nebeneinander liegenden Umlaufkörpern 223, einem gemeinsamen Auffangsteg 224 und einem von einem der Umlaufkörper 223 abragenden Ausleger 222.

Die Umlaufkörper 223 sind im Wesentlichen als gekrümmte Dreiecke geformt. Jeder von ihnen weist eine erste Gleitfläche 223-1, eine spitzwinklig dazu stehende, zweite Gleitfläche 223-2 und eine die beiden vorgenannten Gleitflächen 223-1, 223-2 verbindende, konkav gekrümmte dritte Gleitfläche 223-3 auf. Der in den Figuren 5 und 6 links dargestellte Umlaufkörper 223 weist eine Federnut 225 zur Aufnahme des zweiten Schenkels 242 der Schenkelfeder 24 auf.

Der Auffangsteg 224 ist besagter, konkav gekrümmter, dritter Gleitfläche 223-3 gegenüber angeordnet.

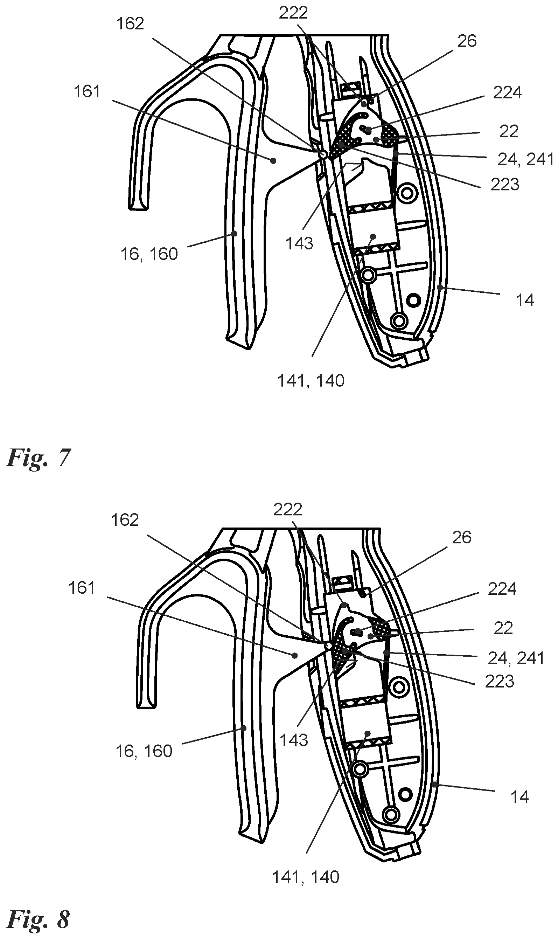

Die Figuren 7 bis 15 zeigen aufeinanderfolgende Schritte des Funktionsablaufs des erfindungsgemäßen Rastmechanismus. Die in Figur 7 gezeigte Stellung entspricht im Wesentlichen derjenigen von Figur 4, wobei jedoch der Labyrinthkörper 22 geschnitten dargestellt ist, wodurch Umlaufkörper 223, Auffangsteg 224, Ausleger 222 und Federzunge 26 klarer unterscheidbar sind. In dieser in Figur 7 gezeigten Initialstellung wird der Labyrinthkörper 22 durch die Kraft der Schenkelfeder 24, von der in den Figuren 7 bis 15 lediglich ihr am Basiselement festgelegter, erster Schenkel 241 sichtbar ist, mit seinem Ausleger 222 gegen die Federzunge 26 gedrückt, die als elastischer Anschlag wirkt und in antagonistischer Wechselwirkung mit der Schenkelfeder 24 die dargestellte Initialstellung des Labyrinthkörpers definiert. In dieser Stellung präsentiert sich der Umlaufkörper 223 dem auf einer geführten Schwenkbewegung anlaufenden Raststift 162 so, dass dieser auf die erste Gleitfläche 223-1 trifft und bei weitergehender Betätigung des Hebels 16 zu einer in Figur 8 gezeigten Nickbewegung des Labyrinthkörpers 22 entgegen der Federkraft der Schenkelfeder 24 führt. Die Federzunge 26 liegt dabei nicht mehr am Ausleger 222 an und übt daher kein Drehmoment auf den Labyrinthkörper 22 aus.

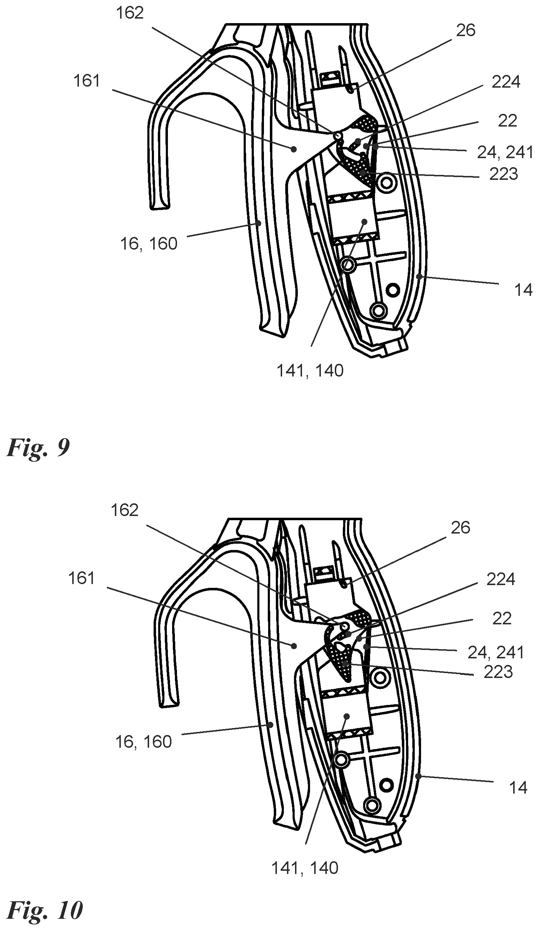

Figur 9 zeigt den Moment, in dem der Raststift 162 das hintere Ende der ersten Gleitfläche 223-1, d.h. die erste Umlaufkörperkante erreicht. Zu diesem Zeitpunkt endet das von dem Raststift 162 auf den Labyrinthkörper 223 ausgeübte Drehmoment und die Schenkelfeder 24 kann sich entspannen, was zu einer Rücknickbewegung des Labyrinthkörpers 22 führt. Dies führt bei einer gleichzeitigen Weiterverschwenkung des Hebels 16 zu einer Kollision des Raststiftes 162 mit dem Auffangsteg 224, wie in Figur 10 dargestellt. In dieser Position befindet sich der Hebel 16 in seiner gehäusenahen bzw. der Mobilteil 160 in seiner basisnahen Endstellung. Bei der konkret dargestellten Ausführungsform gleitet nach der Kollision des Raststifts 162 mit dem Auffangsteg 224, der Auffangsteg 224 beim weiteren Verschwenken des Hebels 16 an der Unterkante des Vorsprungs 161 entlang; dabei hebt sich der Raststift 162 von dem Auffangsteg minimal ab. Es wäre jedoch auch durchaus denkbar, nur den Raststift 162 in Kontakt mit dem Auffangsteg 224 zu lassen.

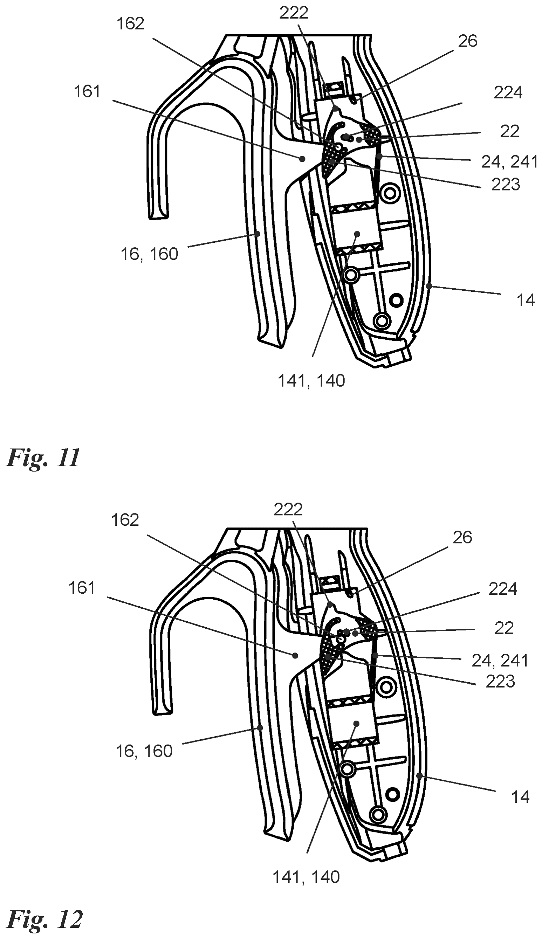

Loslassen des Hebels 16 führt nun dazu, dass, wie in Figur 11 dargestellt, der Raststift 162 an den Scheitelpunkt der konkaven, dritten Gleitfläche 223-3 gleitet, sodass der Hebel 16 mit dem Labyrinthkörper 22 und über diesen mit dem Selektorkörper 14 bzw. das Mobilteil 160 mit dem Basisteil 140 verrastet ist, was eine unmittelbare Rücküberführung des Hebels 16 in seine gehäuseferne bzw. des Mobilteils 160 in seine basisferne Endstellung unterbindet.

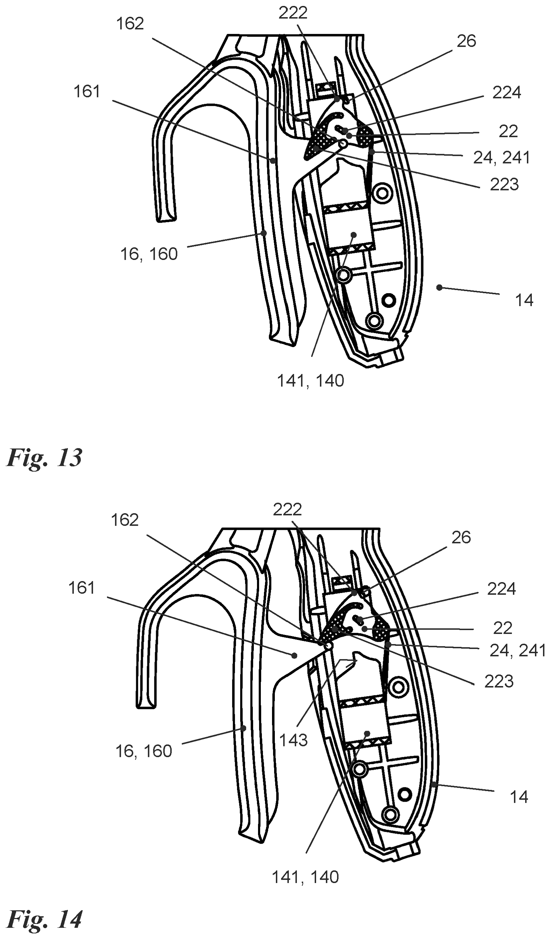

Erst bei erneuter Betätigung des Hebels 16 gleitet der Raststift 162 zur zweiten Umlaufkörperkante, d. h. zum anderen Ende der konkaven, dritten Gleitfläche 223-3 und in den von dieser gemeinsam mit dem gegenüberliegenden Auffangsteg 224 gebildeten Kanal, wie in Figur 12 gezeigt. Hierdurch wird der Raststift 162 über die Kante der dritten Gleitfläche 223-3 gezwungen, sodass der Labyrinthkörper 22, wie in Figur 13 gezeigt, in seine Initialstellung zurückschnappen kann.

Nun ist eine Rücküberführung des Hebels 16 in seine gehäuseferne Endstellung wieder möglich. Dabei gleitet der Raststift 162 an der zweiten Gleitfläche 223-2 entlang und hebt dabei den Labyrinthkörper 22 entgegen der nun wieder einsetzenden Federkraft der Federzunge 26 über die Initialstellung hinaus an. Dies ist in Figur 14 gezeigt.

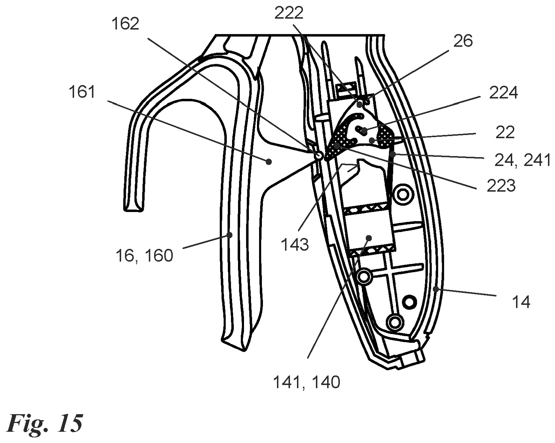

Sobald der Raststift 162 aber von der Spitze des Umlaufkörpers 223 freikommt, wie in Figur 15 gezeigt, drückt die Federzunge 26 den Labyrinthkörper 22 zurück in seine Initialstellung.

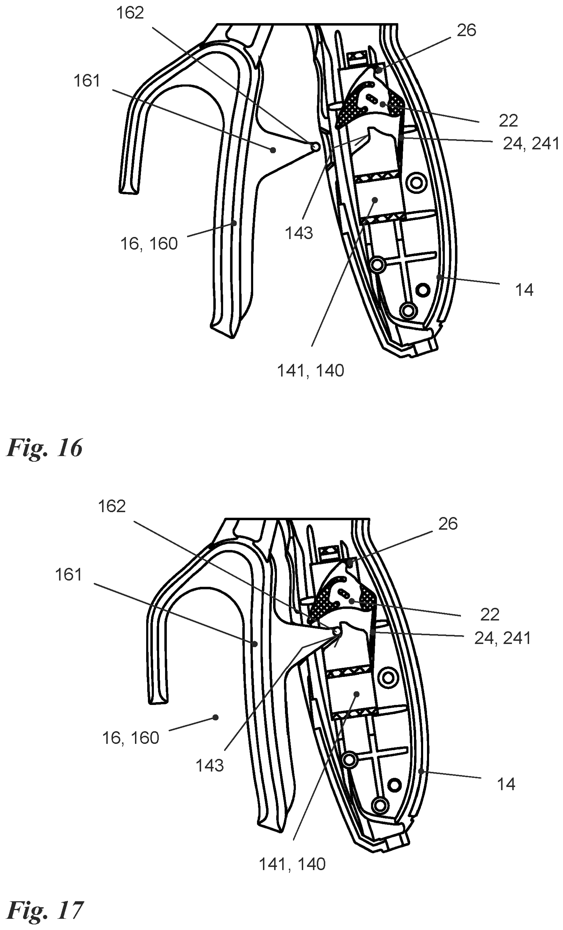

Der oben erläuterte Mechanismus setzt voraus, dass der Labyrinthkörper 22 in seiner Initialstellung so positioniert ist, dass der Raststift 162 mit der Spitze des Umlaufkörpers 223, insbesondere mit dessen erster Gleitfläche 223-1 kollidieren kann. Diese Voraussetzung ist in der "eingeschalteten" Stellung des Selektorkörpers 141 der Fall. In den Figuren 16 und 17 ist die "ausgeschaltete" Stellung des Selektorkörpers 141 gezeigt, in der der Raststift 162 deutlich unterhalb des Labyrinthkörpers 22 entlangläuft. Die gehäusenahe Endstellung des Hebels 16 wird durch einen entsprechenden Anschlag 143 gewährleistet. Eine Wechselwirkung zwischen dem Labyrinthkörper 22 und dem Raststift 162 kommt jedoch nicht zustande, sodass auch die temporäre Rastwirkung, wie oben beschrieben, nicht eintritt.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. So kann im Rahmen der vorliegenden Erfindung alternativ zu dem gezeigten Schwenkhebel ein Schiebehebel 16 Einsatz finden. Weiter ist es denkbar, dass in der "ausgeschalteten" Stellung des Selektorkörpers 141 der Raststift 162 oberhalb des Labyrinthkörpers 22 entlangläuft. Der Selektorkörper kann auch, anders als hier dargestellt, in nicht-linearer Weise, bspw. verkippbar zwischen seinen beiden Stellungen gelagert sein. Auch ist denkbar, dass der Labyrinthkörper 22 in anderer Orientierung angelenkt ist und bei der Wechselwirkung mit dem Raststift 162 anfänglich nicht nach unten, sondern nach oben nickt. Als Alternative zur vorliegenden Erfindung ist es auch denkbar, dass der Labyrinthkörper an dem Mobilelement angebracht und der Rastzapfen drehbar im Basiselement gelagert ist.

### Bezugszeichenliste

- 10: chirurgisches Gerät
- 12: Bedienteil
- 14: Griffgehäuse
- 140: Basiselement
- 141: Selektorkörper
- 142: Schieber
- 143: Anschlag
- 16: Hebel
- 160: Mobilelement
- 161: Vorsprung
- 162: Raststift
- 18: Schaft
- 20: Effektor
- 201: erste Klemmbacke
- 202: zweite Klemmbacke
- 22: Labyrinthkörper
- 221: Achsstutzen
- 222: Ausleger
- 223: Umlaufkörper
- 223-1: erste Gleitfläche
- 223-2: zweite Gleitfläche
- 223-3: dritte Gleitfläche
- 224: Auffangsteg
- 225: Federnut
- 24: Schenkelfeder
- 241: erster Schenkel von 24
- 242: zweiter Schenkel von 24
- 243: Federöse
- 26: Federzunge
- 261: Anlagepunkt von 26

## Patentansprüche

1. Rastmechanismus zur temporären Verrastung eines Mobilelementes (160) mit einem Basiselement (140), wobei das Mobilelement (160) relativ zum Basiselement (140) auf einer vorgegebenen Bewegungsbahn geführt zwischen einer basisfernen Endstellung und einer basisnahen Endstellung bewegbar ist, umfassend
- einen an dem Mobilelement (160) festgelegten und quer zur Bewegungsbahn ausgerichteten Raststift (162) und
- einen bewegbar an dem Basiselement (140) gelagerten Labyrinthkörper (22) mit einem von dem Raststift (162) gleitend umfahrbaren Umlaufkörper (223) mit parallel zu dem Raststift (162) erstreckten Gleitflächen, darunter eine erste Gleitfläche (223-1) und eine zweite Gleitfläche (223-2), die zueinander in einem dem Mobilelement (160) zugewandten, spitzen Winkel stehen und an ihren dem spitzen Winkel abgewandten Enden durch eine konkav geformte, dritte Gleitfläche (223-3), in die die erste Gleitfläche (223-1) über eine erste Umlaufkörperkante und die zweite Gleitfläche (223-2) über eine zweite Umlaufkörperkante übergeht, miteinander verbunden sind,
wobei der Raststift (162) und der Labyrinthkörper (22) ausgelegt sind, derart zusammenzuwirken, dass nach erstmaliger Überführung des Mobilelementes (160) in die basisnahe Endstellung eine Rücküberführung in die basisferne Endstellung blockiert wird und nach nochmaliger Überführung des Mobilelementes (160) in seine basisnahe Endstellung eine Rücküberführung in die basisferne Endstellung wieder zugelassen wird,
und wobei der Labyrinthkörper (22) um eine parallel zu dem Raststift (162) ausgerichtete Schwenkachse schwenkbeweglich an dem Basiselement (140) gelagert ist und die Schwenkachse auf der dem Mobilelement (160) abgewandten Seite der dritten Gleitfläche (223-3) und beabstandet von dieser angeordnet ist,
**gekennzeichnet durch**
zwei antagonistisch auf den Labyrinthkörper einwirkende Federn, deren einander entgegengesetzte Drehmomente den Labyrinthkörper, wenn er sich nicht in Wechselwirkung mit dem Raststift befindet, in einer vorgegebenen Initialstellung halten.

2. Rastmechanismus nach Anspruch 1,
**dadurch gekennzeichnet,**
**dass** sich zwischen dem Basiselement (140) und dem Labyrinthkörper (22) eine Schenkelfeder (24) mit einem ersten Schenkel (241), einem zweiten Schenkel (242) und einer die Schenkel (241, 242) verbindenden Federöse (243) abstützt, wobei die Federöse (243) einen Lagerzapfen der Schwenkachse konzentrisch umläuft, der erste Schenkel (241) am Basiselement (140) festgelegt ist und der zweite Schenkel (242) am Labyrinthkörper (22) festgelegt ist.

3. Rastmechanismus nach Anspruch 2,
**dadurch gekennzeichnet,**
**dass** an dem Basiselement (140) eine Federzunge (26) festgelegt ist, deren freies Ende zumindest in einigen Schwenkpositionen des Labyrinthkörpers (22) an einer Anlagefläche des Labyrinthkörpers (22) anliegt und ein der Federwirkung der Schenkelfeder (24) entgegengerichtetes Drehmoment auf den Labyrinthkörper (22) ausübt.

4. Rastmechanismus nach Anspruch 3,
**dadurch gekennzeichnet,**
**dass** die Federkraft der Federzunge (26) diejenige der Schenkelfeder (24) überwiegt.

5. Rastmechanismus nach einem der vorangehenden Ansprüche,
**dadurch gekennzeichnet,**
**dass** der Labyrinthkörper (22) weiter einen vom Umlaufkörper (223) beabstandeten, der dritten Gleitfläche (223-3) gegenüber liegenden Auffangsteg (224) aufweist.

6. Rastmechanismus nach einem der vorangehenden Ansprüche,
**dadurch gekennzeichnet,**
**dass** der Labyrinthkörper (22) zwei zueinander parallele, voneinander um einen Spalt beabstandete und hinsichtlich der ersten, zweiten und dritten Gleitflächen (223-1, 223-2, 223-3) gleich gestaltete Umlaufkörper (22) umfasst und der Raststift (162) sich beidseitig eines Vorsprungs (161) des Mobilelementes (160) erstreckt, welcher in der basisnahen Endstellung in den Spalt zwischen den Umlaufkörpern (22) eingreift.

7. Rastmechanismus nach den Ansprüchen 5 und 6,
**dadurch gekennzeichnet,**
**dass** den beiden Umlaufkörpern (22) ein gemeinsamer, sich über die gemeinsame Breite der Umlaufkörper (22) und des Spaltes erstreckender Auffangsteg (224) gegenüberliegt.

8. Rastmechanismus nach einem der vorangehenden Ansprüche,
**dadurch gekennzeichnet,**
**dass** das Basiselement (140) ein Selektorkörper (141) ist, der zwischen zwei Selektorpositionen beweglich an einem Basiskörper gelagert ist, relativ zu welchem das Mobilelement (160) geführt bewegbar gelagert ist,
wobei in einer ersten der zwei Selektorpositionen der Labyrinthkörper (22) auf der Bewegungsbahn des Raststiftes (162) und in der zweiten der zwei Selektorpositionen der Labyrinthkörper (22) außerhalb der Bewegungsbahn des Raststiftes (162) liegt.

9. Handgriff für ein chirurgisches Gerät (10), umfassend
- ein Griffgehäuse (14) mit einer Gehäusewandung und einem von der Gehäusewandung umgebenen Gehäuseinnenraum,
- einen relativ zu dem Griffgehäuse (14) verstellbaren Hebel (16) und
- einen Rastmechanismus nach einem der vorangehenden Ansprüche,
wobei das Griffgehäuse (14) einen das Basiselement (140) des Rastmechanismus tragenden Basiskörper und der Hebel (16) das Mobilelement (160) des Rastmechanismus bilden
und wobei der Labyrinthkörper (22) im Gehäuseinneren gelagert und der Raststift (162) auf einem dem Griffgehäuse (14) zugewandten Vorsprung (161) des Hebels (16) angeordnet ist, welcher wenigstens während eines in Richtung der basisnahen Endstellung letzten Abschnitts der Bewegungsbahn durch eine Ausnehmung in der Gehäusewandung in das Gehäuseinnere hineinragt.

## Claims

1. A latching mechanism for temporarily locking a movable element (160) to a base element (140), wherein the movable element (160) is movable relative to the base element (140) along a predetermined path of motion between an end position distant from the base and an end position close to the base, comprising
- a latching pin (162) fixed to the movable element (160) and oriented transversely to the path of motion, and
- a labyrinth body (22) movably mounted on the base element (140), having a circled body (223) that can be slidingly circled by the latching pin (162), with sliding surfaces extending parallel to the latching pin (162), including a first sliding surface (223-1) and a second sliding surface (223-2) that form an acute angle with each other facing the movable element (160) and are connected at their ends opposite the acute angle by a concave third sliding surface (223-3), into which the first sliding surface (223-1) transitions via a first edge of the circled body and the second sliding surface (223-2) transitions via a second edge of the circled body,
wherein the latching pin (162) and the labyrinth body (22) are designed to interact in such a way that, after the movable element (160) has been moved for a first time to the end position close the base, a return to the end position distant from the base is blocked, and after the movable element (160) has been moved once more to its end position close the base, a return movement to the end position distant from the base is permitted,
and wherein the labyrinth body (22) is mounted on the base element (140) such that it can pivot about a pivot axis aligned parallel to the latching pin (162), and the pivot axis is arranged spaced apart from on the third sliding surface (223-3) on its side facing away from the movable element (160),
**characterized by**
two springs acting in opposition on the labyrinth body, whose opposing torques hold the labyrinth body in a predetermined initial position when it is not in contact with the latching pin.

2. The latching mechanism according to claim 1,
**characterized in**
**that** a leg spring (24) with a first leg (241), a second leg (242), and a spring eye (243) connecting the legs (241, 242) is supported between the base element (140) and the labyrinth body (22), wherein the spring eye (243) encircles a bearing journal of the pivot axis concentrically, the first leg (241) is secured to the base element (140), and the second leg (242) is fixed to the labyrinth body (22).

3. The latching mechanism according to claim 2,
**characterized in**
**that** a spring tongue (26) is secured to the base element (140), the free end of which rests against a contact surface of the labyrinth body (22) in at least some of the of the labyrinth body's (22) pivot positions and exerts a torque on the labyrinth body (22) that opposes the spring action of the leg spring (24).

4. The latching mechanism according to claim 3,
**characterized in**
**that** the spring force of the spring tongue (26) exceeds that of the leg spring (24).

5. The latching mechanism according to any one of the preceding claims,
**characterized in**
**that** the labyrinth body (22) further comprises a catchment bridge (224) spaced apart from the circled body (223) and facing the third sliding surface (223-3).

6. The latching mechanism according to any one of the preceding claims,
**characterized in**
**that** the labyrinth body (22) comprises two parallel circled bodies (22) spaced apart by a gap and having the same configuration regarding their respective first, second, and third sliding surfaces (223-1, 223-2, 223-3), the latching pin (162) extending on both sides of a projection (161) of the movable element (160), which, in the end position close to the base, engages in the gap between the circled bodies (22).

7. The latching mechanism according to claims 5 and 6,
**characterized in**
**that** a common catchment bridge (224), extending across the combined width of the circled bodies (22) and the gap, is positioned opposite the two circled bodies (22).

8. The latching mechanism according to any one of the preceding claims,
**characterized in**
**that** the base element (140) is a selector body (141) which is mounted on a base body movably between two selector positions, the movable element (160) being guidedly movably mounted relative to said base body,
wherein, in a first of the two selector positions, the labyrinth body (22) lies on the path of motion of the latching pin (162), and in the second of the two selector positions, the labyrinth body (22) lies outside the path of motion of the latching pin (162).

9. Handle for a surgical device (10), comprising
- a handle housing (14) having a housing wall and an interior space enclosed by the housing wall,
- a lever (16) adjustable relative to the handle housing (14), and
- a latching mechanism according to one of the preceding claims,
wherein the handle housing (14) forms a base body supporting the base element (140) of the latching mechanism, and the lever (16) forms the movable element (160) of the latching mechanism
and wherein the labyrinth body (22) is mounted inside the housing and the latching pin (162) is arranged on a projection (161) of the lever (16) facing the handle housing (14), which projection extends into the interior of the housing through a recess in the housing wall at least during a section of the path of motion final toward the end position close to the base.

## Revendications

1. Mécanisme d'enclenchement destiné à l'enclenchement temporaire d'un élément mobile (160) avec un élément de base (140), l'élément mobile (160) pouvant se déplacer par rapport à l'élément de base (140) selon une trajectoire de mouvement prédéfinie entre une position extrême éloignée de la base et une position extrême proche de la base, comprenant
- une goupille d'encliquetage (162) fixée à l'élément mobile (160) et orientée transversalement par rapport à la trajectoire de mouvement, et
- un corps en labyrinthe (22) monté de manière mobile sur l'élément de base (140), comportant un corps à contournement (223) pouvant être contourné par glissement par la goupille d'encliquetage (162) et présentant des surfaces de glissement s'étendant parallèlement à la goupille d'encliquetage (162), parmi lesquelles une première surface de glissement (223-1) et une deuxième surface de glissement (223-2), qui forment entre elles un angle aigu tourné vers l'élément mobile (160) et qui sont reliées entre elles, à leurs extrémités opposées à cet angle aigu, par une troisième surface de glissement (223-3) de forme concave, dans laquelle la première surface de glissement (223-1) se prolonge par un premier bord du corps à contournement et la deuxième surface de glissement (223-2) par un deuxième bord du corps à contournement,
la goupille d'encliquetage (162) et le corps en labyrinthe (22) étant conçus pour coopérer de telle sorte qu'après un premier déplacement de l'élément mobile (160) vers la position extrême proche de la base, un retour vers la position extrême éloignée de la base soit bloqué et qu'après un nouveau déplacement de l'élément mobile (160) dans sa position extrême proche de la base, un retour vers la position extrême éloignée de la base soit autorisé,
et le corps en labyrinthe (22) étant monté sur l'élément de base (140) de manière à pouvoir pivoter autour d'un axe de pivotement aligné parallèlement à la goupille d'encliquetage (162), cet axe de pivotement étant disposé sur le côté de la troisième surface de glissement (223-3) opposé à l'élément mobile (160) et à distance de celle-ci,
**caractérisé par**
deux ressorts agissant de manière antagoniste sur le corps en labyrinthe, dont les torques opposés maintiennent le corps en labyrinthe dans une position initiale prédéfinie lorsqu'il n'est pas en interaction avec la goupille d'enclenchement.

2. Mécanisme d'enclenchement selon la revendication 1,
**caractérisé en ce**
**qu'**entre l'élément de base (140) et le corps en labyrinthe (22) s'appuie un ressort à branches (24) comportant une première branche (241), une deuxième branche (242) et un œillet (243) reliant les branches (241, 242), l'œillet (243) s'enroulant de manière concentrique autour d'un tourillon de l'axe de pivotement, la première branche (241) étant fixée à l'élément de base (140) et la deuxième branche (242) étant fixée au corps en labyrinthe (22).

3. Mécanisme d'encliquetage selon la revendication 2,
**caractérisé en ce**
**qu'**un ressort à languette (26) est fixée à l'élément de base (140), l'extrémité libre de ce ressort à languette (26) s'appuie, au moins dans certaines positions de pivotement du corps en labyrinthe (22), contre une surface d'appui du corps en labyrinthe (22) et exerce sur ce dernier un torque opposé à l'effet élastique du ressort à branches (24).

4. Mécanisme d'encliquetage selon la revendication 3,
**caractérisé en ce**
**que** la force élastique du ressort à languette (26) l'emporte sur celle du ressort à branches (24).

5. Mécanisme d'enclenchement selon l'une des revendications précédentes,
**caractérisé en ce**
**que** le corps en labyrinthe (22) comporte en outre un pontet de recueil (224) espacée du corps à contournement (223) et située en face de la troisième surface de glissement (223-3).

6. Mécanisme d'encliquetage selon l'une des revendications précédentes,
**caractérisé en ce**
**que** le corps en labyrinthe (22) comprend deux corps à contournement (22) parallèles l'un à l'autre, espacés l'un de l'autre d'un interstice et de forme identique par rapport aux première, deuxième et troisième surfaces de glissement (223-1, 223-2, 223-3) et que la goupille d'encliquetage (162) s'étend de part et d'autre d'une saillie (161) de l'élément mobile (160), laquelle, dans la position extrême proche de la base, s'engage dans l'interstice entre les corps à contournement (22).

7. Mécanisme d'enclenchement selon les revendications 5 et 6,
**caractérisé en ce**
**qu'**un pontet de recueil commun (224), s'étendant sur la largeur totale des corps à contournement (22) et de l'interstice, est disposée en face des deux corps à contournement (22).

8. Mécanisme d'enclenchement selon l'une des revendications précédentes,
**caractérisé en ce**
**que** l'élément de base (140) est un corps de sélection (141) monté de manière mobile entre deux positions de sélection sur un corps de base, par rapport auquel l'élément mobile (160) est monté de manière guidée et mobile,
le corps en labyrinthe (22) se trouvant, dans une première des deux positions de sélection, sur la trajectoire de mouvement de la goupille d'encliquetage (162) et, dans la deuxième des deux positions de sélection, à l'extérieur de la trajectoire de mouvement de la goupille d'encliquetage (162).

9. Poignée pour un instrument chirurgical (10), comprenant
- un boîtier de poignée (14) comportant une paroi de boîtier et un espace intérieur entouré par ladite paroi de boîtier,
- un levier (16) réglable par rapport au boîtier de poignée (14) et
- un mécanisme d'enclenchement selon l'une des revendications précédentes,
le boîtier de poignée (14) formant un corps de base supportant l'élément de base (140) du mécanisme d'encliquetage et le levier (16) formant l'élément mobile (160) du mécanisme d'encliquetage
et dans lequel le corps en labyrinthe (22) est logé à l'intérieur du boîtier et la goupille d'enclenchement (162) est disposée sur une saillie (161) du levier (16) tournée vers le boîtier de poignée (14), laquelle saillie pénètre à l'intérieur du boîtier à travers un évidement dans la paroi du boîtier, au moins pendant une dernière partie de la trajectoire de déplacement en direction de la position extrême proche de la base.
